# DEMANDE DE BREVET EUROPEEN

(11) **EP 1 844 757 A1**
(43) Date de publication de la demande: **17.10.2007**
(21) Numéro de dépôt: 07105740.0
(22) Date de dépôt: 05.04.2007
(51) Int. Cl.: A61K 8/60, A61Q 7/00, A61Q 19/08

(54) **Utilisation de dérivé c-glycosides comme agent protecteur et/ou activateur des lymphocytes gamma delta t**

(30) Priorité: 07.04.2006 FR 0651269
(71) Demandeur: L'Oréal, 75008 Paris (FR)
(72) Inventeur: Breton, Lionel, 78000 Versailles (FR)
(74) Mandataire: Bernstein, Claire Jacqueline

(57) **Abrégé**

La présente invention se rapporte à l'utilisation d'au moins un composé de formule générale (I) : 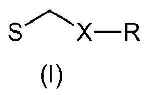 comme agent protecteur et/ou stimulant de l'activité et/ou de la prolifération des cellules lymphocytaires T de type gamma-delta (γδT cells) dans une composition renfermant un milieu cosmétiquement ou pharmaceutiquement acceptable.

La composition trouvera des applications pour favoriser la réparation tissulaire de la peau, pour rééquilibrer les désordres de prolifération et de différenciation épidermique qui apparaissent avec le manque de sommeil, pour améliorer l'aspect de la chevelure et limiter la chute des cheveux.

## Description

La présente invention se rapporte à l'utilisation d'au moins un composé de formule générale (I) : 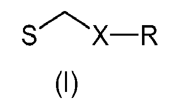 comme agent protecteur et/ou stimulant de l'activité et/ou de la prolifération des cellules lymphocytaires T de type gamma-delta (γδT cells) dans une composition renfermant un milieu cosmétiquement ou pharmaceutiquement acceptable.
La composition trouvera des applications pour favoriser la réparation tissulaire de la peau, pour rééquilibrer les désordres de prolifération et de différenciation épidermique qui apparaissent avec le manque de sommeil, pour améliorer l'aspect de la chevelure et limiter la chute des cheveux.

La peau humaine est constituée d'un compartiment superficiel, l'épiderme et d'un compartiment profond, le derme. L'épiderme est composé principalement, de trois types de cellules qui sont les kératinocytes (majoritaires), les mélanocytes et les cellules de Langerhans. Chacun de ces types cellulaires contribue par ses fonctions propres au rôle essentiel joué dans l'organisme par la peau, notamment le rôle de protection de l'organisme des agressions extérieures. Le derme fournit à l'épiderme un support solide. C'est également son élément nourricier. Il est principalement constitué de fibroblastes et d'une matrice extracellulaire composée elle-même principalement de collagène, d'élastine et d'une substance fondamentale. On y trouve aussi des leucocytes, des mastocytes et des macrophages tissulaires. Enfin, Le derme est traversé par des vaisseaux sanguins et des fibres nerveuses.

La peau constitue une barrière contre les agressions extérieures, notamment chimiques, mécaniques, et à ce titre un certain nombre de réactions de défense contre l'environnement (climat, rayons ultraviolets, tabac, pollutions...) et/ou les xénobiotiques (comme par exemple certains médicaments) se produisent à son niveau.

Chez les sujets en bonne santé, les cellules endommagées par les agressions extérieures sont éliminées par le système immunitaire cutané. Cependant, par exemple lors du manque de sommeil, le système immunitaire est immunodéprimé et ne peut jouer son rôle dans la surveillance de la peau.

Les lymphocytes humains sont caractérisés par un récepteur pour un antigène (TCR, T cell receptor) spécifique. 95% de la population lymphocytaire est constituée de lymphocytes dont les récepteurs de l'antigène des lymphocytes T sont des hétéro dimères transmembranaires composés d'une chaîne *alpha* et *beta* (αβT). Seuls environ 5% des lymphocytes expriment des chaînes gamma et delta (γδT), cette population a été mis en évidence par Tonegawa *et al.* en 1984.
Les lymphocytes γδT sont principalement localisés dans le sang (pour le sous-type Vδ2) et dans les épithélia : épiderme et muqueuses (pour le sous-type résident Vδ1). Ils expriment les récepteurs CD3 et les récepteurs TCR gamma-delta (respectivement avec deux régions variables en fonction de leur localisation circulante : Vγ9Vδ2 ou résidante : Vγ9Vδ1) et sont souvent CD4(-) - CD8(-), leur activation n'est pas restreinte au complexe majeur d'histocompatibilité (CMH) et on leur attribue un rôle dans la régulation de l'homéostasie cellulaire. La chaîne gamma est codée par le chromosome 7 et la chaîne delta par le 14.
Les lymphocytes γδT constituent une population lymphocytaire T dont l'analyse des caractéristiques structurales a modifié les connaissances sur les interactions entre la cellule T et son antigène. L'importance des fonctions immunorégulatrices exercées par ces cellules ouvre de nouvelles perspectives dans la compréhension des mécanismes qui concourent au maintien de l'homéostasie du système immunitaire. Les lymphocytes γδT semblent intervenir très précocement dans la régulation de l'immunité innée.

De récents travaux démontrent que ces cellules T spécifiques jouent un rôle important dans l'homéostasie de l'épiderme (Nature Immunology, 6,1 2005, 73-76). En effet, les lymphocytes γδT de la peau et/ou des muqueuses induisent la sécrétion et la libération d'IGF1 dans la peau, cette libération d'IGF1 conduit à un contrôle de l'homéostasie épidermique notamment, l'équilibre entre prolifération et différenciation des cellules épidermiques.

La peau subit un remodelage constant, certaines de ces transformations observées avec le temps, sont notamment, la résultante d'une modification de la sécrétion naturelle de l'organisme en hormones (hormones de croissance, prolactine, oestrogène, etc...) et en facteurs de croissance (TGFα et TGFβ, EGF, IGF, VEGF...).

Le rôle de ces hormones et/ou de ces facteurs de croissance est d'autant plus important qu'avec le temps, leur libération et leur impact sur les tissus cibles diminuent, la croissance des tissus se stabilise, tandis que la dégradation matricielle qui s'amplifie, n'est plus compensée par l'action de régénération de ces facteurs hormonaux et/ou de croissance.

Parmi ces facteurs de croissance, les IGFs (Insulin Growth Factors) jouent un rôle prépondérant. Ces facteurs ont été découverts lors de l'exploration du mécanisme d'action de l'hormone de croissance GH (Growth Hormone), qui stimule la croissance de tous les tissus, y compris cutanés. L'hormone de croissance (GH) est constituée de 191 acides aminés liés en une séquence spécifique et est sécrétée par la partie antérieure de la glande hypophyse, cette sécrétion peut être renforcée par l'exercice physique ainsi que par d'autres facteurs. Le rôle biologique de la GH est fondamental, non seulement pour la croissance staturale d'un organisme jeune, mais également pour le maintien de son intégrité à l'âge adulte. La GH agit sur les organes périphériques et le cerveau soit directement, soit indirectement en stimulant la synthèse des facteurs de croissance, tels les *insulin-like growth factors* (IGF I et II) ou l'*epidermal growth factor* (EGF) ou celle de leurs récepteurs. L'action directe de la GH est de type anti-insulinique en favorisant la lipolyse au niveau des tissus adipeux.
Par l'intermédiaire de l'IGF1, la GH stimule l'incorporation des acides aminés dans les protéines, la croissance des cartilages et des os (croissance staturale) et la prolifération cellulaire de nombreux organes, dont la peau.

L'hormone de croissance et les autres hormones somatotropes, telle la somatomédine C (ou IGF1) sont aptes à maintenir son aspect jeune au corps. Elles interviennent, en effet, dans la gestion de son métabolisme, déterminant non seulement la taille finale du corps adulte mais donnant encore, volume, tonus et fermeté aux organes et tissus, particulièrement aux muscles. En fait, l'hormone de croissance participe à tout ce qui procure une bonne image de soi et un mental positif. Elle raffermit le corps et rend le dos plus droit, développe les muscles des épaules et du bassin. Elle diminue également la graisse, surtout au ventre, augmente la libido et l'énergie sexuelle, la repousse et la coloration des cheveux, ainsi que l'élasticité de la peau. De façon peut-être moins apparente mais tout aussi bénéfique, ses effets se constatent dans une meilleure résistance à l'effort, un sommeil moins prolongé et plus profitable, une pression artérielle équilibrée, une meilleure acuité visuelle, auditive et cérébrale.

Chez tous les mammifères étudiés, y compris l'homme, la GH est sécrétée de façon pulsatile et ce caractère constitue un facteur déterminant pour un grand nombre d'effets biologiques de l'hormone.

Les causes du déclin de GH lié à l'âge sont mal connues. Chez l'homme, à partir de la puberté, on observe une diminution de la sécrétion de GH de l'ordre de 10 % tous les dix ans. Au cours du vieillissement, la perte de masse musculaire, l'accumulation de tissu adipeux, la déminéralisation osseuse, la perte de la capacité de régénération tissulaire sont concomitantes avec la diminution de la sécrétion de GH. Cette dernière favorise un rapport catabolisme sur anabolisme accru entraînant ainsi une situation de déséquilibre qui aggrave les effets du vieillissement.
Des facteurs additionnels, comme la prise de poids, voire l'obésité, liée à l'âge, les taux d'hormones stéroïdiennes, le manque de sommeil ou un certain niveau de résistance tissulaire à l'action de la GH peuvent également être importants.
Parallèlement, il existe une diminution notable de la qualité du sommeil avec l'âge (diminution du sommeil à ondes lentes, du sommeil paradoxal et augmentation des périodes et de la durée de l'éveil interrompant les phases de sommeil). Le premier phénomène du vieillissement est une baisse marquée du sommeil profond (slow wave sleep ou SWS), qui peut survenir aussi tôt qu'à l'âge de 36 ans et qui est remplacé par un sommeil plus léger. Le passage de la quarantaine à l'âge avancée est ensuite associé à une baisse de la quantité de sommeil et de la durée de la phase paradoxale (rapid eye movement ou REM) et du sommeil profond (non-REM). Les statistiques démontrent que la population affectée par les troubles du sommeil totalise 93 millions en Amérique du Nord, en Europe et au Japon.

Il est concevable que le manque de sommeil ou la baisse qualitative du sommeil liée à l'âge, contribue aux modifications hormonales et à leurs conséquences métaboliques. En effet, des traitements pharmacologiques tendant à augmenter le sommeil à ondes lentes entraînent également une augmentation de la sécrétion de GH.

Le déficit en hormone de croissance se traduit par des symptômes physiques : perte des cheveux, cheveux fins, lèvres et ensemble maxillaire amincis, peau déshydratée, ventre pendant, coussinets de graisse au niveau des genoux... et des symptômes psychologiques :
fatigue permanente, difficultés à contrôler ses émotions, épuisement après un effort physique, faible estime de soi, dépression...

Les taux d'hormones de croissance circulant dans le sang stimulent la production, à partir du foie, d'une autre hormone, l'IGF1 (*Insuline like Growth Factor 1),* dont le rôle de médiateur permet à l'hormone de croissance de développer ses effets positifs. La mesure du taux d'IGF1, également appelé Somatomédine C, est considérée comme plus sûre que celle de la GH, pratiquement indétectable de jour chez l'homme. Le foie est le plus important site de production des IGFs mais de nombreuses cellules sont capables de produire des IGFs. Deux types sont classiquement décrits : IGF1 et IGF2. Ce sont deux peptides dont la séquence en acide aminé s'apparente à celle de l'insuline, d'où leur nom. Il existe deux récepteurs, respectivement pour l'IGF1 et pour l'IGF2. Le récepteur à l'IGF1 présente une affinité partagée avec l'insuline. Ce n'est pas le cas pour le récepteur à l'IGF2.
Avec le vieillissement, le taux des IGFs, diminue pour se stabiliser à l'âge adulte : il s'agit de la somatopause (D. Radman « Effects of human growth hormone in men over 60 yeard old »; N. Engl J. Med 1990, Juls ; 323(1) : 1-6*).*

Il est connu dans l'état de la technique que l'IGF1 seul stimule la prolifération des kératinocytes *(*Neely EK- Insulin-like growth factors are mitogenic for human keratinocytes and a squamous cell carcimona - J Invest Dermatol 1991 Jan ; 96(1) :104-10*).* En ce qui concerne les fibroblastes, il est également connu que l'IGF1 stimule la synthèse des GAGs, et la synthèse du collagène. Par ailleurs, des études ont mis en évidence une participation non négligeable des IGFs dans la cicatrisation. Enfin, une étude réalisée *in vivo* chez l'homme a démontré qu'un traitement percutané par IGF1 pendant un mois se traduisait par une augmentation de l'épaisseur cutanée.
Il a aussi été démontré que la diminution de l'expression d'IGF1 était associée à la chute accrue des cheveux (Tang et al. 2003, J. Am. Acad. Dermatol. Aug;49(2):229-33).

La demanderesse a mis en évidence que les composés de formule générale (I): 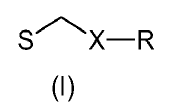 stimule et/ou protège les cellules γδT et qu'ainsi cet extrait peut induire la sécrétion et la libération d'IGF1 dans la peau. Ce contrôle de la libération d'IGF1 participe au maintien de l'homéostasie épidermique qui régule notamment, l'équilibre entre prolifération et différenciation des cellules épidermiques.

Ainsi, selon un premier de ses objets, la présente invention se rapporte à l'utilisation d'au moins un composé de formule générale (I) dans laquelle, 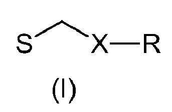
- S représente un monosaccharide ou un polysaccharide jusqu'à 20 unités sucre, sous forme pyranose et/ou furanose et de série L et/ou D, ledit mono- ou polysaccharide présentant au moins une fonction hydroxyle obligatoirement libre et/ou éventuellement une ou plusieurs fonctions amine éventuellement protégée ;
- la liaison S-CH₂X représente une liaison de nature C-anomèrique;
- X représente un groupement choisi parmi: -CO-, -CH(NR₁R₂)-, -CHR'-, -C(=CHR')- ;
- R représente une chaîne alkyle, perfluoroalkyle, hydrofluoroalkyle linéaire ou ramifiée, saturée ou insaturée, un cycle cycloalkyle, cycloperfluoroalkyle, cyclohydrofluoroalkyle, comprenant de 1 à 18 atomes de carbone, un radical phényle ladite chaîne, ledit cycle ou ledit radical pouvant être éventuellement interrompu par un ou plusieurs hétéroatomes choisi parmi l'oxygène, le souffre, l'azote, le silicium, et éventuellement substituée par au moins un radical choisi parmi -OR'₁, -SR"₁, -NR"'₁R'₂, -COOR"₂, -CONHR"'₂, -CN, halogène, perfluoroalkyle, hydrofluoroalkyle et/ou au moins un radical cycloalkyle, aryle, hétérocyclique éventuellement substitués ;
- R', R₁, R₂, identiques ou différents ont la même définition que celle donnée pour R, et peuvent également représenter un hydrogène et un radical hydroxyle ;
- R'₂, R'''₂, identiques ou différents, représentent un atome d'hydrogène, un radical choisi parmi un radical alkyle, hydroxyle, perfluoroalkyle et/ou hydrofluoroalkyle, linéaire ou ramifié, saturé ou insaturé, comprenant de 1 à 20 atomes de carbone ;
- R'₁, R"₁, R"₂, R"'₁, identiques ou différents, représentent un atome d'hydrogène, un radical choisi parmi un radical alkyle, perfluoroalkyle et/ou hydrofluoroalkyle, linéaire ou ramifié, saturé ou insaturé, comprenant de 1 à 20 atomes de carbone;
   avec les restrictions suivantes :

- R₁ et R₂ ne peuvent pas être simultanément un radical hydroxyle ;
- R'''₁ et R'₂ ne peuvent pas être simultanément un radical hydroxyle ;
- quand S est un D-xylose et X est =CO alors R ne peut pas être un phenyl non substitué ;
- quand S est un D-glucose et X est la fonction -OH alors R ne peut pas être un p-hydroxyphenyl ; et
- quand S est un D-glucose et X est =O alors R ne peut pas être un p-methoxy-phenyl ou un hexyle
   pour maintenir et/ou rétablir l'équilibre entre prolifération et différentiation des cellules épidermiques.

Les composés C-glycosides utilisables selon l'invention représentent une sous famille des dérivés C-glycosides décrits dans l'EP 1 345 919, ils peuvent être préparés selon le procédé décrit dans ce document.

De façon préférée, les composés de formule générale (I) utilisés selon l'invention sont tels que :
- S représente un monosaccharide ou un polysaccharide jusqu'à 5 unités sucre, sous forme pyranose et/ou furanose et de série L et/ou D, ledit mono- ou polysaccharide présentant au moins une fonction hydroxyle obligatoirement libre et/ou éventuellement une ou plusieurs fonctions amine éventuellement protégée ;
- R représente une chaîne alkyle, linéaire ou ramifiée, saturée ou insaturée, un cycle cycloalkyle, comprenant de 1 à 14 atomes de carbone, un radical phényle, (la dite chaîne, ledit cycle ou ledit radical pouvant être éventuellement substituée par au moins un radical choisi parmi -OR'₁, -NR"'₁R'₂, -COOR"₂, -CONHR"'₂;
- R', R₁, R₂, identiques ou différents ont la même définition que celle donnée pour R, et peuvent également représenter un hydrogène et un radical hydroxyle ;
- R'₂, R'"₂, identiques ou différents, représentent un atome d'hydrogène, un radical choisi parmi un radical alkyle, hydroxyle, linéaire ou ramifié, saturé ou insaturé, comprenant de 1 à 8 atomes de carbone ;
- R'₁, R"₁, R"₂, R"'₁, identiques ou différents, représentent un atome d'hydrogène, un radical choisi parmi un radical alkyle, linéaire ou ramifié, saturé ou insaturé, comprenant de 1 à 8 atomes de carbone ;
   la définition des autres substituantsde la formule générale (I) restant inchangée.

Selon un autre variante, on préférera les composés de formule générale (I) tels que:
- S représente un monosaccharide ou un polysaccharide jusqu'à 2 unités sucre, sous forme pyranose et/ou furanose et de série L et/ou D, ledit mono- ou polysaccharide présentant au moins une fonction hydroxyle obligatoirement libre et/ou éventuellement une ou plusieurs fonctions amine éventuellement protégée ;
- X représente un groupement choisi parmi: -CO-, -CH(NR₁R₂)-, -CHR'
   la définition des autres substituants de la formule générale (I) restant inchangée ou adoptant les valeurs préférées ci-dessus.

Avantageusement, les monosaccharides préférés sont choisis parmi le D-glucose, le D-mannose, le D-xylose, le D-lyxose, L-arabinose, le L-rhamnose, l'acide D-glucuronique, l'acide D-galacturonique, l'acide D-iduronique, la N-acétyl-D-glucosamine, la N-acétyl-D-galactosamine et désigne avantageusement le D-glucose, le D-xylose, la N-acétyl-D-glucosamine ou le L-fucose, et très préférentiellement le D-xylose.

Avantageusement encore, les polysaccharides préférés contenant jusqu'à 6 unités sucre sont choisis parmi le D-maltose, le D-cellobiose, le D-maltotriose, un disaccharide associant un acide uronique choisi parmi l'acide D-iduronique ou l'acide D-glucuronique avec une hexosamine choisi parmi la D-galactosamine, la D-glucosamine, la N-acétyl-D-galactosamine, la N-acétyl-D-glucosamine, un oligosaccharide contenant au moins un xylose avantageusement choisis parmi le xylobiose, le méthyl-β-xylobioside, le xylotriose, le xylotétraose, le xylopentaose et le xylohexaose et préférentiellement le xylobiose qui est composé de deux molécules de xylose liés par une liaison 1-4.

Parmi les dérivés C-glycosides de formule (I) utilisés selon l'invention, on préfère tout particulièrement :
Composé 1. 1-(C-β-D-xylopyranosyl)-propane-2-one 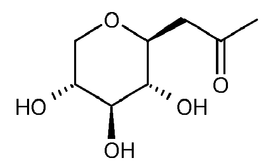
Composé 2. 1-(C-β-D-glucopyranosyl)-propane-2-one

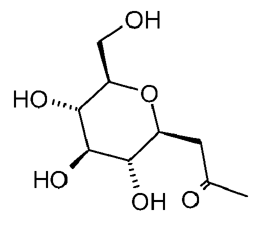

L'utilisation d'un agent protecteur et/ou stimulant des lymphocytes γδT de la peau et/ou des muqueuses conduit à la libération accrue d'IGF1 par les cellules épidermiques et favorise la prolifération physiologique des keratinocytes et/ou diminue la différenciation épidermique.

Compte tenu du fait que la libération de l'IGF1 est dépendante de la sécrétion de GH et de prolactine et que ces hormones sont libérées de façon pulsatile lors de la phase d'endormissement et uniquement lors de la phase d'endormissement (il s'agit d'une alternance veille/sommeil et non d'une alternance jour/nuit), la présente invention concerne également l'utilisation cosmétique d'au moins un composé de formule générale (I) pour mimer l'effet du sommeil sur le renouvellement cellulaire épidermique et, ainsi, compléter l'effet du sommeil sur la peau ou supplémenter le dysfonctionnement des fonctions épidermiques cutanées qui peuvent apparaître lors d'une absence de sommeil.

L'utilisation selon l'invention visera donc plus particulièrement à mimer les effets du sommeil sur le renouvellement cellulaire épidermique pour prévenir et/ou corriger les effets cutanés caractéristiques du manque de sommeil et/ou à stimuler la peau lors de l'absence de sommeil et induire le renouvellement des cellules épidermiques.
Ainsi l'utilisation selon l'invention permet de prévenir et/ou de traiter les manifestations cutanées générées par le ralentissement du renouvellement des cellules, conduit ainsi à la régénération cellulaire de l'épiderme et améliore l'apparence de la surface de la peau.
Les conséquences de cette activité est que l'utilisation des composés de formule générale (I) selon l'invention sur un sujet en manque de sommeil permet plus particulièrement de traiter les traits tirés et/ou creusés, en particulier autour des yeux, d'uniformiser le teint.

Selon un second objet, la présente invention se rapporte à un procédé de traitement cosmétique pour effacer les signes cutanés de la fatigue en stimulant la régénération des cellules épidermiques caractérisé en ce qu'il comprend l'application sur le visage d'au moins un composé de formule générale (I).

L'utilisation d'au moins un composé de formule générale (I) permet aussi de prévenir et/ou traiter des désordres capillaires tels qu'une modification de la densité, de la quantité ou de la qualité des cheveux, conséquence par exemple d'un ralentissement, d'un arrêt de la croissance ou d'une chute des follicules pileux.

Ainsi selon un autre de ses objet, la présente invention se rapporte à l'utilisation d'au moins un composé de formule générale (I) pour prévenir l'amincissement de la fibre kératinique et/ou induire la pousse de cheveux et/ou des poils ; pour induire la repousse de cheveux ou de poils plus denses.

Aussi, l'invention se rapporte encore à l'utilisation d'au moins un composé de formule générale (I) comme agent pour induire et/ou stimuler la croissance des fibres kératiniques, cheveux ou poils en particulier humains et/ou freiner leur chute et/ou augmenter leur densité.

Par augmenter la densité des fibres kératiniques, et notamment la densité capillaire, on entend augmenter le nombre de fibres kératiniques, notamment de cheveux, par cm² de peau telle que le cuir chevelu.

Ainsi, une autre utilisation d'au moins un composé de formule générale (I) se rapporte aux compositions de traitement capillaire (shampooing, lotion, masques...) pour limiter et/ou éviter la chute des cheveux et ainsi traiter l'alopécie de quelque nature qu'elle soit et/ou favoriser la pousse de cheveu sain.

L'utilisation cosmétique selon l'invention de composé de formule générale (I) peut se faire à l'aide d'une composition cosmétique de soin et/ou de maquillage des fibres kératiniques.

L'invention s'applique aussi aux fibres kératiniques des mammifères de l'espèce animale (chien, cheval ou chat par exemple).

Les fibres kératiniques humaines auxquelles s'applique l'invention sont notamment les cheveux, les sourcils, les cils, les poils de barbe et de moustache. Plus spécialement, l'invention s'applique aux cheveux et/ou aux cils humains.

La présente invention a également pour objet un procédé de traitement cosmétique des fibres kératiniques humaines et/ou de la peau d'où émergent les dites fibres, y compris le cuir chevelu, destiné notamment à stimuler la croissance des fibres kératiniques humaines telles que les cheveux et les cils d'être humain et/ou freiner leur chute, caractérisé par le fait qu'il consiste à appliquer sur les fibres kératiniques humaines et/ou la peau d'où émergent les dites fibres, une composition cosmétique comprenant une quantité efficace d'au moins un composé de formule générale (I), à laisser celle-ci en contact avec les fibres kératiniques et/ou la peau d'où émergent les dites fibres, et éventuellement à rincer les fibres kératiniques et/ou ladite peau.
Ce procédé de traitement présente les caractéristiques d'un procédé cosmétique dans la mesure où il permet d'améliorer l'esthétique des fibres kératiniques et en particulier des cheveux et des cils en leur donnant une plus grande vigueur et un aspect amélioré. En outre, il peut être utilisé quotidiennement pendant plusieurs mois, sans prescription médicale. Plus spécialement, la présente invention a pour objet un procédé de soin cosmétique des cheveux et/ou du cuir chevelu humain, en vue d'améliorer leur état et/ou leur aspect, caractérisé en ce qu'il consiste à appliquer sur les cheveux et/ou le cuir chevelu, une composition cosmétique comprenant au moins un composé de formule générale (I), à laisser celle-ci au contact des cheveux et/ou du cuir chevelu, et éventuellement à rincer les cheveux et/ou le cuir chevelu.

L'invention a encore pour objet un procédé de soin cosmétique et/ou de maquillage ces cils humains, en vue d'améliorer leur état et/ou leur aspect, caractérisé en ce qu'il consiste à appliquer une composition de mascara comprenant au moins un composé de formule générale (I) et à laisser celle-ci au contact des cils. Cette composition de mascara peut être appliquée seule ou en sous-couche d'un mascara pigmenté classique et être éliminée comme un mascara pigmenté classique.

Les compositions utilisées selon l'invention peuvent être administrées par voie orale, entérale ou encore par voie topique, on préférera l'administration par voie topique.

Dans le cas d'une administration par voie orale, les compositions peuvent se présenter sous toute forme adaptée telle qu'une solution buvable, des gélules, dragée, capsule molle ou dure, comprimés à avaler ou à croquer, granulés à dissoudre, sirop, aliment solide ou liquide...

La composition peut aussi se présenter sous les formes galéniques classiquement utilisées pour une application topique et notamment sous forme de dispersions du type lotion ou sérum, d'émulsions de consistance liquide ou semi-liquide du type lait, obtenues par dispersion d'une phase grasse dans une phase aqueuse (H/E) ou inversement (E/H), ou de suspensions ou émulsions de consistance molle, semi-solide ou solide du type crème ou gel, ou encore d'émulsions multiples (E/H/E ou H/E/H), de microémulsions, de nanoémulsions, de dispersions vésiculaires de type ionique et/ou non ionique, ou des dispersions cire/phase aqueuse. Ces compositions sont préparées selon les méthodes usuelles.
Elle peut également se présenter sous la forme d'un système transdermique permettant une libération active ou passive du(des) actif(s) par transdermie, par exemple de type patch ou gel patch (hydrogel).

La composition utilisée selon l'invention peut ainsi constituer une composition de traitement ou de soin de la peau (y compris le cuir chevelu), des fibres kératiniques (cheveux, cils, sourcils), des ongles ou des lèvres, ou une composition de protection solaire ou de bronzage artificiel, ou encore un produit nettoyant ou démaquillant de la peau, des cheveux, des sourcils ou des cils, un produit déodorant ou encore un composé parfumant. Elle est alors généralement non colorée ou faiblement colorée, et elle peut contenir éventuellement des actifs cosmétiques ou dermatologiques. Elle peut alors être utilisée comme base de soin pour la peau ou les lèvres (baumes à lèvres, protégeant les lèvres du froid et/ou du soleil et/ou du vent), comme crème de soin de jour ou de nuit pour la peau du visage et/ou du corps. Elle peut, en outre, se présenter sous forme de shampooing traitant ou non, colorant ou non, et d'après-shampooing.

La composition utilisée selon l'invention peut également constituer une composition cosmétique colorée et notamment une composition de maquillage de la peau, des fibres kératiniques (cheveux ou cils) et/ou des muqueuses, en particulier un fond de teint, un blush, un fard à joues ou à paupières, un composé anti-cernes en stick, un rouge à lèvres ou un brillant à lèvres, présentant éventuellement des propriétés de soin ou de traitement. De préférence, il pourra s'agir d'une composition de maquillage colorée (beige ou verte) destinée à corriger la couleur du teint.

Selon la destination de la composition utilisée selon l'invention, elle pourra également comprendre des actifs cosmétiques ou dermatologiques qui seront choisis par l'homme du métier de telle sorte qu'ils ne nuisent pas à l'effet des composés de formule générale (I) selon l'invention.

Dans le cadre de l'utilisation selon l'invention pour prévenir et/ou traiter des désordres capillaires, les compositions seront à usage cosmétique et en particulier d'application topique sur la peau et les fibres kératiniques, et plus spécialement sur le cuir chevelu, les cheveux et les cils, elles peuvent se présenter sous toutes les formes galéniques connues adaptées au mode d'utilisation, par exemple, celles mentionnées plus haut.

En particulier, la composition à application sur le cuir chevelu ou les cheveux peut se présenter sous forme d'une lotion de soin capillaire, par exemple d'application journalière ou bihebdomadaire, d'un shampooing ou d'un après-shampooing capillaire, en particulier d'application bi-hebdomadaire ou hebdomadaire, d'un savon liquide ou solide de nettoyage du cuir chevelu d'application journalière, d'un produit de mise en forme de la coiffure (laque, produit pour mise en pli, gel coiffant), d'un masque traitant, d'une crème ou d'un gel moussant de nettoyage des cheveux. Elle peut encore se présenter sous forme de teinture ou de mascara capillaire à appliquer au pinceau ou au peigne.

Par ailleurs, pour une application sur les cils ou les poils, la composition à laquelle s'applique l'invention peut se présenter sous forme d'un mascara, pigmenté ou non, à appliquer à la brosse sur les cils ou encore sur les poils de barbe ou de moustache.

Selon un mode de réalisation particulier, la composition selon l'invention se présente sous forme de crème ou lotion capillaire, de shampooing ou d'après-shampooing capillaire, de mascara capillaire ou pour cils.

Selon un mode particulier de réalisation de l'invention, on peut associer au composé de formule générale (I) au moins un composé actif capillaire additionnel favorisant la repousse et/ou limitant la chute des fibres kératiniques et notamment des cheveux.

Ledit actif capillaire pourra être choisi parmi :
- les anti-séborrhéiques tels que certains acides aminés soufrés, l'acide 13-cis rétinoïque, l'acétate de cyprotérone ;
- les agents de lutte contre les états squameux du cuir chevelu (pellicules) comme le zinc pyrithione, le disulfure de sélénium, le climbazole, l'acide undécylénique, le Kétoconazole, la piroctone olamine (octopirox) ou la ciclopiroctone (ciclopirox);
- les actifs stimulant la repousse et/ou favorisant le ralentissement de la chute des cheveux, on peut plus particulièrement citer à titre non limitatif :
   * les esters d'acide nicotinique, dont notamment le nicotinate de tocophérol, le nicotinate de benzyle et les nicotinates d'alkyles en C₁-C₆ comme les nicotinates de méthyle ou d'hexyle ;
   * les dérivés de pyrimidine, comme le 2,4-diamino 6-piperidinopyrimidine 3-oxyde ou "Minoxidil" décrit dans les brevets US 4,139,619 et US 4,596,812 ; l'Aminexil ou 2,4 diamino pyrimidine 3 oxyde décrit dans WO96/09048 ;
   * les agents à la fois inhibiteurs de la lipoxygénase et inducteurs de la cyclo-oxygénase, ou les agents inducteurs de la cyclo-oxygénase favorisant la repousse des cheveux comme ceux décrits par la Demanderesse dans la demande de brevet européen EP 0 648 488 ;
- les agents antibiotiques tels que les macrolides, les pyranosides et les tétracyclines, et notamment l'Erythromycine ;
- la Cinnarizine, la Nimodipine et la Nifedipine;
- des hormones, telles que l'estriol ou des analogues, ou la thyroxine et ses sels;
- des agents antiandrogènes, tels que l'oxendolone, la spironolactone, le diéthylstilbestrol et la flutamide ;
- la cromakalim et le nicorandil.
   La composition selon l'invention peut être appliquée sur les zones alopéciques du cuir chevelu et des cheveux d'un individu, et éventuellement laissée en contact plusieurs heures et éventuellement rincée.

### Exemple 1 - Mise en évidence de l'induction de la prolifération des lymphocytes γδT par des dérivés C-glycosides de formule générale (I)

L'activité d'induction de la prolifération des lymphocytes γδT est testée de la façon suivante: des cellules de sang périphérique humain sont mises en culture en présence d'un milieu de culture de type RPMI supplémenté par de la L-Glutamine (2mM), de la pénicilline/streptomycine (50µg/50Ui/ml), et du sérum de veau foetal (10%). Les dérivés C-glycosides 1 et 2 sont ajoutés à différentes concentrations (10 à 0.05mM) ainsi que la phytohemagglutine (PHA à 5 *G/ml), contrôle positif de la prolifération lymphocytaire totale. Après 3 jours de culture la prolifération est révélée par un marquage au BrdU.

Les résultats obtenus sont les suivants :

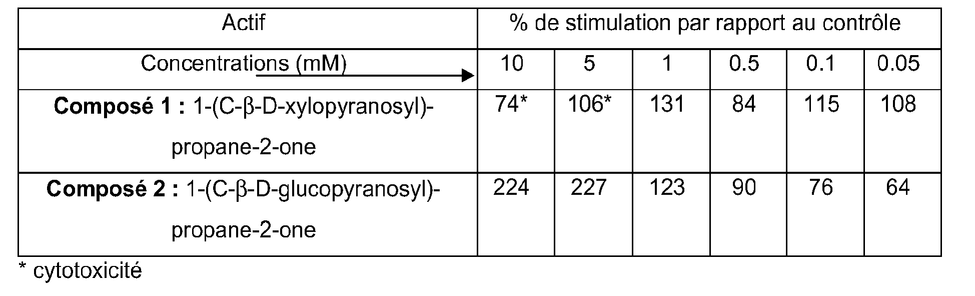

Tous les dérivés testés présentent une forte capacité de prolifération des lymphocytes humains à partir de 1 mM.

### EXEMPLE 2 : Compositions selon l'invention

### Lotion démaquillante pour le visage

| | | |
|---|---|---|
| Composé 2 | | 1,00 |
| Chlorure de strontium | | 5,00 |
| Antioxydant | | 0,05 |
| Isopropanol | | 40,00 |
| Conservateur | | 0,30 |
| Eau | qsp | 100 %. |

### Gel pour le soin du visage

| | | |
|---|---|---|
| Composé 1 | | 5,00 |
| Eau thermale de Vichy | | 10,00 |
| Polymère épaississant | | 1,00 |
| Antioxydant | | 0,05 |
| Isopropanol | | 40,00 |
| Conservateur | | 0,30 |
| Eau | qsp | 100 %. |

### Crème réparatrice « Sommeil »

| | | |
|---|---|---|
| Composé 1 | | 1,00 |
| Stéarate de glycérol | | 2,00 |
| Polysorbate 60 (Tween 60 vendu par la société ICI) | | 1,00 |
| Acide stéarique | | 1,40 |
| Triéthanolamine | | 0,70 |
| Carbomer | | 0,40 |
| Fraction liquide du beurre de karité | | 12,00 |
| Perhydrosqualène | | 12,00 |
| Antioxydant | | 0,05 |
| Conservateur | | 0,30 |
| Eau | qsp | 100 % |

### Mascara cire/eau

- Cire d'abeilles 6,00 %
- Cire de paraffine 13,00 %
- Huile de jojoba hydrogénée 2,00 %
- Polymère filmogène hydrosoluble 3,00 %
- Stéarate de triéthanolamine 8,00 %
- composé 2 1,00 %
- Pigment noir 5,00 %
- Conservateur qs
- Eau qsp 100,00 %

Ce mascara s'applique sur les cils comme un mascara classique avec une brosse à mascara.

## Revendications

1. Utilisation d'au moins un composé de formule générale (I) : 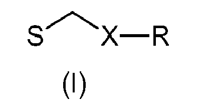 dans laquelle,
- S représente un monosaccharide ou un polysaccharide jusqu'à 20 unités sucre, sous forme pyranose et/ou furanose et de série L et/ou D, ledit mono- ou polysaccharide présentant au moins une fonction hydroxyle obligatoirement libre et/ou éventuellement une ou plusieurs fonctions amine éventuellement protégée ;
- la liaison S-CH₂X représente une liaison de nature C-anomèrique ;
- X représente un groupement choisi parmi: -CO-, -CH(NR₁R₂)-, -CHR'-, -C(=CHR')- ;
- R représente une chaîne alkyle, perfluoroalkyle, hydrofluoroalkyle linéaire ou ramifiée, saturée ou insaturée, un cycle cycloalkyle, cycloperfluoroalkyle, cyclohydrofluoroalkyle, comprenant de 1 à 18 atomes de carbone, un radical phényle la dite chaîne, ledit cycle ou ledit radical pouvant être éventuellement interrompu par un ou plusieurs hétéroatomes choisi parmi l'oxygène, le souffre, l'azote, le silicium, et éventuellement substituée par au moins un radical choisi parmi -OR'₁, -SR"₁, -NR"'₁R'₂, -COOR"₂, -CONHR"'₂, -CN, halogène, perfluoroalkyle, hydrofluoroalkyle et/ou au moins un radical cycloalkyle, aryle, hétérocyclique éventuellement substitués ;
- R', R₁, R₂, identiques ou différents ont la même définition que celle donnée pour R, et peuvent également représenter un hydrogène et un radical hydroxyle ;
- R'₂, R"'₂, identiques ou différents, représentent un atome d'hydrogène, un radical choisi parmi un radical alkyle, hydroxyle, perfluoroalkyle et/ou hydrofluoroalkyle, linéaire ou ramifié, saturé ou insaturé, comprenant de 1 à 20 atomes de carbone ;
- R'₁, R"₁, R"₂, R"'₁, identiques ou différents, représentent un atome d'hydrogène, un radical choisi parmi un radical alkyle, perfluoroalkyle et/ou hydrofluoroalkyle, linéaire ou ramifié, saturé ou insaturé, comprenant de 1 à 20 atomes de carbone;
avec les restrictions suivantes :
- R₁ et R₂ ne peuvent pas être simultanément un radical hydroxyle;
- R"'₁ et R'₂ ne peuvent pas être simultanément un radical hydroxyle;
- quand S est un D-xylose et X est =CO alors R ne peut pas être un phenyle non substitué ;
- quand S est un D-glucose et X est la fonction -OH alors R ne peut pas être un p-hydroxyphenyle; et
- quand S est un D-glucose et X est =O alors R ne peut pas être un p-methoxy-phenyle ou un hexyle
pour maintenir et/ou rétablir l'équilibre entre prolifération et différentiation des cellules épidermiques.

2. Utilisation selon la revendication 1, **caractérisée en ce que** les composés de formule générale (I) sont tels que S représente un monosaccharide ou un polysaccharide jusqu'à 5 unités sucre, sous forme pyranose et/ou furanose et de série L et/ou D, ledit mono- ou polysaccharide présentant au moins une fonction hydroxyle obligatoirement libre et/ou éventuellement une ou plusieurs fonctions amine éventuellement protégée.

3. Utilisation selon la revendication 1 ou 2, **caractérisée en ce que** les polysaccharides préférés contenant jusqu'à 6 unités sucre sont choisis parmi le D-maltose, le D-cellobiose, le D-maltotriose, un disaccharide associant un acide uronique choisi parmi l'acide D-iduronique ou l'acide D-glucuronique avec une hexosamine choisi parmi la D-galactosamine, la D-glucosamine, la N-acétyl-D-galactosamine, la N-acétyl-D-glucosamine, un oligosaccharide contenant au moins un xylose avantageusement choisis parmi le xylobiose, le méthyl-β-xylobioside, le xylotriose, le xylotétraose, le xylopentaose et le xylohexaose et préférentiellement le xylobiose qui est composé de deux molécules de xylose liés par une liaison 1-4.

4. Utilisation selon la revendication 2, **caractérisée en ce que** S représente un monosaccharide ou un polysaccharide jusqu'à 2 unités sucre, sous forme pyranose et/ou furanose et de série L et/ou D, ledit mono- ou polysaccharide présentant au moins une fonction hydroxyle obligatoirement libre et/ou éventuellement une ou plusieurs fonctions amine éventuellement protégée.

5. Utilisation selon la revendication 4, **caractérisée en ce que** les monosaccharides préférés sont choisi parmi le D-glucose, le D-mannose, le D-xylose, le D-lyxose, L-arabinose, le L-rhamnose, l'acide D-glucuronique, l'acide D-galacturonique, l'acide D-iduronique, la N-acétyl-D-glucosamine, la N-acétyl-D-galactosamine et désigne avantageusement le D-glucose, le D-xylose, la N-acétyl-D-glucosamine ou le L-fucose, et très préférentiellement le D-xylose.

6. Utilisation selon l'une quelconque des revendications précédentes, **caractérisée en ce que** R représente une chaîne alkyle, linéaire ou ramifiée, saturée ou insaturée, un cycle cycloalkyle, comprenant de 1 à 14 atomes de carbone, un radical phényle, ladite chaîne, ledit cycle ou ledit radical pouvant être éventuellement substituée par au moins un radical choisi parmi -OR'₁, -NR'"₁R'₂, -COOR"₂, -CONHR'"₂.

7. Utilisation selon l'une quelconque des revendications précédentes, **caractérisée en ce que** le composé de formule générale (I) est choisi parmi :
Compose 1. 1-(C-β-D-xylopyranosyl)-propane-2-one ;
Composé 2. 1-(C-β-D-glucopyranosyl)-propane-2-one.

8. Utilisation selon l'une quelconque des revendications précédentes, pour mimer les effets du sommeil sur le renouvellement cellulaire épidermique.

9. Utilisation selon la revendication 8, pour corriger les effets cutanés provoqués par le manque de sommeil.

10. Utilisation selon la revendication 8 ou 9, pour induire le renouvellement des cellules épidermiques.

11. Utilisation selon l'une quelconque des revendications 8 à 10, pour induire la régénération cellulaire de l'épiderme.

12. Utilisation selon l'une quelconque des revendications 8 à 11, pour améliorer l'apparence de la surface de la peau et/ou traiter les traits tirés ou creusés et/ou uniformiser le teint.

13. Utilisation selon l'une quelconque des revendications 1 à 7, pour prévenir l'amincissement de la fibre kératinique et/ou induire la pousse de cheveux et/ou des poils.

14. Utilisation selon la revendication 13, pour induire la repousse de cheveux ou de poils plus denses.

15. Utilisation selon l'une quelconque des revendications 1 à 7, pour prévenir et/ou traiter la chute des cheveux et/ou des poils.

16. Utilisation selon la revendication 15, pour prévenir et/ou traiter l'alopécie.

17. Utilisation selon l'une quelconque des revendications 13 à 16, **caractérisée en ce que** le ou les composé de formule générale (I) est associé avec au moins un actif choisi parmi les agent anti-chute et/ou activateur de la repousse des cheveux et de poils.

18. Procédé de traitement cosmétique pour effacer les signes cutanés de la fatigue en stimulant la régénération des cellules épidermiques **caractérisé en ce qu'**il comprend l'application sur le visage d'au moins un composé de formule générale (I) selon l'une quelconque des revendications 1 à 7.

19. Procédé de traitement cosmétique des fibres kératiniques humaines et/ou de la peau d'où émergent les dites fibres, y compris le cuir chevelu, destiné à stimuler la croissance des fibres kératiniques humaines et/ou freiner leur chute, **caractérisé par le fait qu'**il consiste :
- à appliquer sur les fibres kératiniques humaines et/ou la peau d'où émergent les dites fibres, une composition cosmétique comprenant au moins un composé de formule générale (I) selon l'une quelconque des revendications 1 à 7,
- à laisser celle-ci en contact avec les fibres kératiniques et/ou la peau d'où émergent les dites fibres, et
- optionnellement à rincer les fibres kératiniques et/ou ladite peau.
